# EUROPÄISCHE PATENTANMELDUNG

(11) **EP 1 195 176 A2**
(43) Veröffentlichungstag der Anmeldung: **10.04.2002**
(21) Anmeldenummer: 01124061.1
(22) Anmeldetag: 09.10.2001
(51) Int. Cl.: A61N 5/067

(54) **Dermatologisches Handstück**

(30) Priorität: 09.10.2000 DE 10049778
(71) Anmelder: TuiLaser AG, 82110 Germering (DE)
(72) Erfinder: Weigand, Claus, 81667 München (DE); Massow, Cornelia, 80636 München (DE)
(74) Vertreter: Alber, Norbert, Dipl.-Ing.

(57) **Zusammenfassung**

Dermatologisches Handstück für die Bestrahlung von lebender Haut mit Behandlungslicht aus einer vom Handstück entfernten Lichtquelle, das einerseits eine schnelle und exakte Positionierung des Handstückes, also Bestimmung der momentanen Behandlungsfläche, zulässt, und insbesondere auch eine Vergrößerung der einzelnen, durch das Handstück bewältigbaren, Behandlungsfläche zulässt, insbesondere jeweils bei definiert bekannter Bestrahlungsintensität der Haut, mit einer Kontaktfläche am vorderen Ende des Handstückes zum Kontaktieren der zu behandelnden Haut, einem Austrittsende der Laserstrahl-Führungsvorrichtung im Handstück und Abstandshalter zwischen dem Austrittsende und der Kontaktfläche, wobei der Abstandshalter den Strahl des Behandlungslichts wenigstens zwischen Austrittsende und Kontaktfläche allseitig umgibt und aus einem für das Behandlungslicht nicht durchlässigen, jedoch für wenigstens ein Orientierungslicht mit anderer Wellenlänge, insbesondere Tageslicht, durchlässigen Material besteht.

## Beschreibung

### I. Anwendungsgebiet

Die Erfindung betrifft ein Handstück einer Behandlungsvorrichtung, mittels der Hautbereiche durch Bestrahlung mit Licht behandelt werden. Die Lichtquelle befindet sich dabei von dem möglichst leicht ausgebildeten Handstück entfernt, und ist mit dem Handstück über eine Licht-Führungsvorrichtung, die in der Regel Licht-Leitfasern umfasst, verbunden.

### II. Technischer Hintergrund

Es ist bekannt, dass bestimmte Hautkrankheiten mittels Bestrahlung durch Licht einer bestimmten Wellenlänge oder bestimmter Wellenlängenbereiche gelindert oder gar geheilt werden können, beispielsweise kann Psoriasis mittels Ultraviolett-Strahlung behandelt werden.

Diese UV-Strahlung wird bisher mittels Fotolampen schmalbandig oder breitbandig, jedoch in nicht kohärenten Wellenlängenbereichen, appliziert. Aufgrund der relativ geringen Strahlungsintensität dieser Lampen dauert die Behandlung lang, und nicht zu bestrahlende Hautbereiche müssen umständlich abgedeckt werden. Auch die auf die einzelnen Hautbereiche aufgebrachten Strahlungsdosen können nie genau ermittelt werden, da nur die Menge des pro Zeiteinheit von der Bestrahlungslampe abgegebenen Lichts, aber nicht der Anteil des hiervon auf der zu behandelnden Hautpartie auftreffenden Lichts, ermittelt werden kann.

Weiterhin ist es bekannt die Haut durch Behandlung mittels kohärentem Licht einer Wellenlänge, insbesondere Laserlicht, zu behandeln. Aufgrund der hohen Energiedichte von Laserlicht wird dies bisher hauptsächlich für die Entfernung von Tätowierungen, Entfernung von Haarwurzeln und Feuermalen u. ä. punktuellen oder kleinflächigen Behandlungen eingesetzt, bei denen im Grunde bestimmt Hautschichten partiell gezielt zerstört werden.

Inzwischen wird Laserlicht auch zur nicht-zerstörenden Behandlung von Hautflächen eingesetzt, beispielsweise mit einem Excimer-Laser von 308 nm als Lichtquelle. Da keine Hautschichten zerstört werden sollen, kommt es bei den hochenergetischen Laserstrahlen ganz besonders auf exakte Dosierung sowie Positionierung - unter Vermeidung von Fehlstellen und Überlappungen - der einzeln nacheinander bestrahlten kleinen Flächenbereiche an, die mittels Laserstrahl behandelt werden sollen.

Ein weitere Nachteil dabei ist die unter Umständen ebenfalls lange Behandlungsdauer, da bei großflächigen Behandlungen sehr viele einzelne Behandlungsflächen nacheinander bestrahlt werden müssen, wobei der größte Zeitaufwand im jeweils erneuten exakten Positionieren des Behandlungsgerätes, also exakten Festlegen der neuen Behandlungsfläche gegenüber den bisherigen oder zukünftigen Behandlungsflächen, besteht.

### III. Darstellung der Erfindung

### a) Technische Aufgabe

Es ist daher die Aufgabe gemäß der vorliegenden Erfindung, ein Handstück zu schaffen, das einerseits eine schnelle und exakte Positionierung des Handstückes, also Bestimmung der momentanen Behandlungsfläche, zulässt, und insbesondere auch eine Vergrößerung der einzelnen, durch das Handstück bewältigbaren, Behandlungsfläche zulässt, insbesondere jeweils bei definiert bekannter Bestrahlungsintensität der Haut.

### b) Lösung der Aufgabe

Diese Aufgabe wird durch die Merkmale der Ansprüche 1, 2 und 21 gelöst. Vorteilhafte Ausführungsformen ergeben sich aus den Unteransprüchen.

In diesem Zusammenhang muss darauf hingewiesen werden, dass folgendes sich bei Hautbehandlungen mittels Laser vor, während oder nach ihrer Bestrahlung als vorteilhaft erwiesen hat, aufgrund der starken auftretenden Erwärmung der Hautfläche durch das Laserlicht:
- Der Auftrag von pastösen bzw. flüssigen Substanzen, die einerseits Kühlung bewirken kann und andererseits Spätfolgen der Erwärmung vermindern kann;
- Das Aufbringen von Flüssigkeit auf der Behandlungsfläche vor ihrer Bestrahlung, deren Brechungsindex dem der Haut ähnlich oder identisch ist, was den Absorptionsgrad der Haut erhöhen kann, also die Intensität der Behandlung erhöhen bzw. die benötigte Bestrahlungsdauer senken kann;
- Eine Druckbelastung der zu behandelnden Hautpartien während der Behandlung, da hierdurch ein Zusammendrücken des Höhenaufbaus der Hautschichten erzielt wird aufgrund dessen ein tieferes Eindringen der Behandlungsstrahlung in die Epidermis erreicht wird; und
- die Strahlungsintensität von Laserstrahlen, auch bei Excimer-Lasern, innerhalb des Strahlquerschnitts variiert und deshalb in der Vergangenheit versucht wurde, z. B. mittels optischer Elemente wie mikrooptisch strukturierter Flächen im Strahlengang eine Egalisierung der Strahlungsintensität über den Strahlquerschnitt zu erreichen, beispielsweise durch Aufteilung der Strahlung in eine Vielzahl von Teilstrahlen.

Durch die erfindungsgemäße Materialwahl des Abstandshalters und dessen rohrförmig geschlossenen Querschnitt wird einerseits eine Abschirmung der Umgebung gegenüber der behandelnden Strahlung erreicht, und andererseits bleibt dennoch die Einsehbarkeit der Behandlungsfläche voll erhalten.

Durch die entsprechende Materialwahl bei der Blende bleibt nicht nur die Behandlungsfläche, sonder auch deren Umgebung einsehbar, was insbesondere die Positionierung einer neuen Behandlungsfläche gegenüber in vorherigen Schritten bereits behandelten Fläche erleichtert.

Durch das Vorhandensein eines Abstandshalters zwischen dem Austrittsende z. B. einer Lichtleitfaser als Strahlführungsvorrichtung und der Behandlungsfläche wird prinzipiell zunächst ein Divergieren des Laserstrahles ermöglicht, und kann durch optische Elemente noch verstärkt werden.

Durch Vergrößern des Abstandes zwischen Austrittsende und Kontaktfläche, beispielsweise durch Verlängern oder Verkürzen der wirksamen Länge des Abstandshalters z. B. durch Verlagern des Austrittsendes entlang des Abstandshalters kann die Größe des auf der Kontaktfläche auftreffenden Strahlquerschnitts variiert werden, wenn auch unter gleichzeitiger gegenläufiger Variation der Bestrahlungsintensität pro Flächeneinheit.

Gleiches kann zwar auch mit Hilfe optischer Elemente erreicht werden, jedoch bedeutet dies in der Regel einen höheren baulichen und damit auch höheren Kostenaufwand, und zum anderen ist dabei ein Rückschluss bzw. eine sofortige Umrechnung in die entsprechende Bestrahlungsintensität pro Flächeneinheit weniger einfach als bei Veränderung des wirksamen Abstandes, der unmittelbar ablesbar und sensorisch auch leicht abnehmbar ist, und damit auch als Eingangsgröße für eine Steuerung der Vorrichtung zur Verfügung steht.

Die Veränderung, insbesondere Vergrößerung, des Strahlquerschnitts an der Kontaktfläche des Handstückes zur Haut hin erleichtert und beschleunigt vor allem die Behandlung großer Hautflächen, ohne die exakte Bestrahlung kleinerer restlicher Hautflächen zu behindern.

Gewöhnlich müssen, z. B. bei Psoriasis, größere, hinsichtlich ihrer Form jedoch ungleichmäßig konturierte, Hautflächen behandelt werden.

Mit dem erfindungsgemäßen Handstück ist dies auf einfache Art und Weise möglich, indem z. B. zuerst die mittigen, großflächigen Bereiche bestrahlt werden durch einzelne aneinandergesetzte, große Behandlungsflächen, also mit maximal an der Kontaktfläche aufgeweitetem Strahlquerschnitt.

Wegen der abnehmenden Strahlungsintensität pro Flächeneinheit muss dabei die Strahlungsdauer pro Behandlungsfläche zwar erhöht werden, wegen der wesentlich geringeren Anzahl von Umsetzungen und Neupositionierungen des Handstückes wird insgesamt dennoch eine relativ hohe Flächenleistung pro Zeiteinheit ermöglicht.

Die verbleibenden kleinflächigen Randbereiche werden anschließend behandelt, indem hierfür der Strahlquerschnitt an der Kontaktfläche auf das gewünschte Maß verkleinert wird und damit die so eingestellte kleinere, beispielsweise in mehreren Schritten verkleinerte, Behandlungsfläche exakt auf den noch nicht behandelten Restflächen der Haut, zum Bestrahlen positioniert werden kann. Durch die minimierte Anzahl von Umsetzungen des Handstückes wird auch die Gefahr von Fehlstellen oder unerwünschten Überlappungen von Behandlungsflächen minimiert.

Die vorbeschriebene Vorgehensweise zur Behandlung der Zentralbereiche der zu behandelnden Hautflächen durch große Behandlungsflächen und anschließende Behandlung der kleineren restlichen Randbereiche mittels kleiner Behandlungsflächen erfordert auch eine eindeutige Markierung bereits behandelter Hautpartien, die auch nach einiger Zeit, beispielsweise 30 Minuten, noch sichtbar sein muss.

Vorzugsweise wird die Behandlungsfläche mittels des Handstücks durch ein Blende am vorderen Ende des Handstückes begrenzt, wobei die Blendenöffnung die Behandlungsfläche definiert.

Durch Verändern der Blendenöffnung kann die Behandlungsfläche von einer maximalen Größe aus verringert werden, ohne die Bestrahlungsintensität pro Flächeneinheit zu verändern. Wenn zusätzlich die Bestrahlungsintensität verändert, beispielsweise erhöht, werden soll, kann gleichzeitig der Querschnitt des auf der Blende, also der Behandlungsfläche, auftreffenden Strahles des Behandlungslichts, insbesondere Laserlichts, verringert werden, beispielsweise durch Verringern der Strahldivergenz oder durch Verringern des Abstandes zwischen dem Austrittsende der Lichtleitvorrichtung und der Blende im Handstück.

Zusätzlich können in der Blende mehrere andere Funktionen untergebracht werden:

Zum Zwecke der vorerwähnten Markierung bereits behandelter Hautflächen kann die Blende ausgebildet sein zum Aufbringen einer Markierungsflüssigkeit oder Markierungsfarbe auf der behandelten Haut um die Blendenöffnung, also um die behandelte Fläche herum. In diesem Fall ist die Blende im die Blendenöffnung umlaufenden Randbereich ganz oder teilweise beispielsweise als Stempel oder auf andere Art und Weise mit Austrittsöffnungen für eine Markierungsflüssigkeit ausgestattet.

Eine andere Möglichkeit besteht darin, nicht die Umgebung, also den umgebenden Rand, oder auch nur die Eckpunkte des umgebenden Randes zu markieren, sondern die zu behandelnde oder behandelte Fläche selbst.

Eine Behandlungssubstanz muss dann im Bereich der Blendenöffnung aufgebracht werden. Dies ist beispielsweise möglich durch Versprühen oder Verspritzen einer flüssigen oder pastösen Markierungssubstanz aus Austrittsöffnungen heraus, die sich im Randbereich der Blende um die Blendenöffnung herum, insbesondere in den der Blendenöffnung zugewandten Flanken der Blende, befindet.

Eine solche Anordnung eröffnet auch die Möglichkeit, anstelle einer reinen Markierungssubstanz eine Behandlungssubstanz aufzubringen, die kühlende, oder anderweitige medizinische Wirkungen entfalten soll. Eine aufgebrachte Substanz, insbesondere Flüssigkeit, kann natürlich auch beide Zwecke, also die der Behandlung und die der Markierung, in sich vereinen, indem beispielsweise eine Behandlungssubstanz entsprechend eingefärbt ist zur Verbesserung der Sichtbarkeit und mit ausreichenden Haftungseigenschaften gegenüber der Haut ausgestattet wird.

Auch ein nachträgliches Verlaufen einer solchen, auch der Markierung dienenden, Substanz nach Abheben des Handstückes von der Haut darf nicht stattfinden, da die Beibehaltung exakter Umfangsgestaltung wesentlich ist für das Ansetzen einer späteren, benachbarten Behandlungsfläche, bei der ja der behandelnde das Handstück so auf der Haut aufsetzt, dass die Blendenöffnung unmittelbar an die Kante einer bereits auf der Haut als behandelt markierten Fläche ansetzt.

Wird die direkt auf der Behandlungsfläche, also in der Blendenöffnung, aufgebrachte Substanz auch zur Markierung benutzt, so ist eine zusätzliche Markierung durch die der Hautfläche zugewandte Blendenunterseite in der Umgebung der Blendenöffnung verzichtbar.

Wie bereits angesprochen, kann das Handstück mehrere Veränderungsmöglichkeiten enthalten:

Zum Einen die Vergrößerung oder Verkleinerung der Blendenöffnung der Blende, wobei vorzugsweise die geometrische Form der Blendenöffnung, also Rechteck, Sechseck oder andere nahtlos aneinander anreihbare geometrische Formen, beibehalten werden sollte. Auch ein Wechsel zwischen solchen aneinander anreihbaren geometrischen Formen ist möglich, beispielsweise eine rechteckige Grundform bei maximaler Blendenöffnung, die bei zunehmender Verkleinerung zunächst in ein Trapez und letztendlich in ein Dreieck übergeht.

Weiterhin kann am Handstück die wirksame Länge des Handstückes, also der Abstand zwischen dem Austrittsende des Laserlichts aus der Strahlführungsvorrichtung, die insbesondere aus Lichtleitfasern besteht, und der Kontaktfläche, also der Blendenöffnung am vorderen Ende des Handstückes, verändert werden. Zu diesem Zweck ist das Austrittsende vorzugsweise in einem Haltekörper, vorzugsweise in Querrichtung mittig, fixiert, und diese Haltevorrichtung kann relativ zum übrigen Handstück in Längsrichtung verstellt werden.

Die Außenwandung des Handstückes, in der Regel ein Rohrabschnitt, dient dabei gleichzeitig als Abstandshalter und als Grundkorpus des Handstückes, und in dessen Inneren kann der Haltekörper in Längsrichtung verstellt, beispielsweise stufenlos verschraubt, werden.

Dabei kann der Haltekörper eine Markierung, beispielsweise in Form eines umlaufenden Ringes, aufweisen und der aus z. B. durchsichtigem Acryglas bestehende, tubusförmige umgebende Abstandshalter eine in Längsrichtung verlaufende Skala aufweisen, an der unmittelbar die durch die momentane Abstandseinstellung bewirkte Strahlungsintensität pro Flächeneinheit oder gar direkt die sich hieraus ergebende Bestrahlungsdauer aufgetragen ist.

Weiterhin sind zur Beeinflussung der Strahldivergenz im Strahlengang des Handstückes, also zwischen Austrittsende und Blendenöffnung, optische Mittel zur Regulierung der Strahldivergenz, beispielsweise eine Streulinse, angeordnet. Durch Beeinflussung dieser optischen Elemente, insbesondere Verlagerung in Längsrichtung bzgl. des Austrittsendes, kann wiederum die Divergenz des Strahles und damit auch die Bestrahlungsintensität pro Flächeneinheit in einem bestimmte Abstand stromabwärts der optischen Einheit, die die Divergenz verändert, beeinflusst werden.

Bei Ablesen der Bestrahlungsintensität am Tubus des Abstandshalters muss dies berücksichtigt werden. Dies könnte beispielsweise dadurch geschehen, dass das Verschieben des optischen Elementes in seinem Abstand gegenüber dem Austrittsende gleichzeitig ein Verschieben der Markierung in gleichem Maß oder mit entsprechendem Übersetzungsverhältnis in Längsrichtung entlang des Haltekörpers bewirkt.

Jedwede Berechnung oder Anzeige einer bestimmten Bestrahlungsintensität auf den bestrahlten Hautflächen, also im Bereich der Blendenöffnung, geht von einer bestimmten Energiedichte am Austrittsende der Laserstrahl-Führungsanordnungen im Handstück aus.

In der Praxis variiert diese Energiedichte jedoch, beispielsweise abhängig von Radius und Anzahl der Biegungen der Lichtleitfasern, die als Laserstrahl-Führungsvorrichtung verwendet werden, oder von deren Alter.

Zur Berücksichtigung derartiger Veränderungen kann im Handstück selbst eine Energiemessung vorgenommen werden, die vorzugsweise ständig im Strahlengang im Handstück angeordnet ist, und bei bzw. vor jeder Bestrahlung die tatsächlich vorhandene Energiedichte des Strahles am Austrittsende mißt und entweder automatisch in die Berechnung von Bestrahlungsdauer etc. einbezieht oder ein Warnsignal abgibt, wenn die gemessene Energiedichte von einer unterstellten Energiedichte zu stark abweicht.

Eine Möglichkeit besteht darin, innerhalb des Handstückes im Strahlengang, vorzugsweise unmittelbar nach dem Austrittsende oder auch nahe vor der Blendenöffnung, mittels eines optischen Elementes, etwa eines halbdurchlässigen Spiegels, einen geringen Anteil von beispielsweise 5% des Laserlichts aus dem Strahlengang in Richtung Blendenöffnung abzulenken und dadurch auszukoppeln und auf einen Meßkopf zur Bestimmung der Energiedichte zu richten.

Sowohl das optische Element als auch der Meßkopf können beispielsweise fest am Haltekörper, welcher auch das Endstück der Führungsvorrichtung für den Laserstrahl aufnimmt, fest angeordnet sein. Der Meßkopf steht natürlich über entsprechende Signalleitungen mit der Steuereinheit des Gerätes oder wenigstens einer optischen Anzeige, die auch am Handstück angeordnet sein kann, in Verbindung.

Vorzugsweise ist die Blende in konstruktiver Hinsicht zwar Bestandteil des Abstandshalters, also insbesondere des tubusförmigen Grundkörpers, demgegenüber jedoch auswechselbar gestaltet.

Durch Auswechseln der Blende können beispielsweise Blenden mit unterschiedlich großer und auch unterschiedlich positionierter (zentral oder randseitig) Blendenöffnung wahlweise eingesetzt werden, wodurch jedoch nur eine stufenweise Veränderung der Blendenöffnung erzielbar ist.

Weiterhin kann durch möglichst einfaches Auswechseln der Blende am Handstück die Blende - auch bei unveränderter Größe der Blendenöffnung - bei jedem Patienten ausgewechselt werden, also als Einmalteil dienen. Wenn zusätzlich sichergestellt ist, dass die zu behandelnde Haut ausschließlich mit der Blende des Handstückes in Kontakt gerät, ist hierdurch eine ausreichende Behandlungshygiene (gegen eine Übertragbarkeit von Keimen vom einem Patienten auf den anderen) allein durch das Auswechseln der Blende sichergestellt, so dass umfangreiche und risikobehaftete Reinigungsarbeiten zwischen der Behandlung zweier Patienten entfallen können.

Die Befestigung der Blende im Handstück erfolgt vorzugsweise formschlüssig. Darüber hinaus kann die Blende gegenüber dem Handstück, insbesondere gegenüber dem Abstandshalter, als insbesondere in Längsrichtung bewegliches Schaltelement ausgebildet sein.

Durch ausreichendes Andrücken der Blende gegenüber dem restlichen Handstück, also durch Druck mittels des Handstückes auf die Haut des Patienten, wird ein Signal ausgelöst, welches den ausreichenden Druck für die Behandlung erkennen lässt. Das Signal kann beispielsweise ein optisches oder akustisches Signal sein, oder auch die Freischaltung der Quelle für das Behandlungslicht bewirken. Auslösender Parameter für das Signal kann entweder der beim Aufdrücken des Handstückes erzeugte Druck sein oder der sich beim Aufdrücken auf die Haut des Patienten verändernde elektrische Widerstand zwischen Handstück und Patient.

Das Behandlungslicht, also,die eigentliche Bestrahlung, wird vom Behandelnden vorzugsweise durch einen Handschalter in Gang gesetzt, der sich vorzugsweise direkt am Handstück befindet, welches ja vom Behandelnden in der Hand, vorzugsweise in der teilweise geschlossenen Faust, gehalten werden soll.

Ein Einstellelement zum Einstellen der Behandlungsdauer pro Positionierung des Handstückes wird entweder an einem separaten Regler vorgenommen, der sich vorzugsweise nicht am Handstück, sondern an der Steuereinheit des Behandlungsgerätes, also vom Handstück entfernt, befindet, oder automatisch aufgrund der vom Handstück mittels Sensoren ermittelten Verstellparameter ermittelt.

Da die Veränderung der Blendenöffnung keine Veränderung der Bestrahlungsintensität pro Flächeneinheit der Haut bewirkt, schlägt sie sich auch nicht in einer veränderten Behandlungsdauer pro Positionierung des Handstückes nieder. Weiterhin kann der Vorratsbehälter für die Behandlungsflüssigkeit und/oder Markierungsflüssigkeit im Inneren des Handstückes untergebracht sein. Befindet sich der Vorratsbehälter außerhalb der Blende, beispielsweise im Inneren des Abstandshalters, so ist eine Schlauchverbindung o. ä. vom Vorratsbehälter ins Innere der Blende, um die Austrittsöffnungen der Blendenaußenfläche zu versorgen, notwendig.

In einer bevorzugten Ausführungsform kann das Innere der Blende direkt als Vorratsbehälter genutzt werden.

In beiden Fällen kann der hierfür notwendige Druck zum Auspressen der Substanz aus den Austrittsöffnungen z. B. konventionell, beispielsweise mittels einer kleinen elektrisch betriebenen Pumpe, aufgebracht werden. In diesem Fall kann sich auch der Vorratsbehälter völlig außerhalb des Handstückes, beispielsweise bei der Steuereinheit oder der Behandlungslichtquelle, befinden, um zusammen mit dem Behandlungslicht über eine Schlauchleitung zugeführt werden.

In einer bevorzugten Ausführungsform befindet sich jedoch der Vorratsbehälter am oder im Handstück und wird mechanisch durch den Behandler mit Druck beaufschlagt, in dem der Anpressdruck der Blende gegenüber dem Handstück gleichzeitig zum Erzeugen des notwendigen Druckes im Vorratsbehälter für die Markierungs- bzw. Behandlungssubstanz benutzt wird.

Dabei kann mit Erzeugen des Druckes, also Anpressen des Handstückes auf der Haut des Patienten, mit Druckerzeugung gleichzeitig das Ausspritzen der Substanz auf die Behandlungsfläche erfolgen, oder zeitverzögert, indem ein separates Öffnungsventil, welches die Austrittsöffnungen freigibt, von Hand oder mittels Steuerung zum gewünschten Zeitpunkt (vor, während oder nach Bestrahlung mit dem Behandlungslicht) freigegeben wird. Zu diesem Zweck ist das Ventil von Hand betätigbar oder an die Steuerung des gesamten Gerätes angeschlossen.

Sofern das Innere der Blende selbst als Vorratsbehälter verwendet wird, hat dies den Vorteil, dass die Blende somit eine Menge an Behandlung bzw. Markierungssubstanz enthält, die für eine bestimmte Anzahl von Positionierungen, also Behandlungen, des Handstückes ausreicht, die bei einem einzigen Patienten relativ selten überschritten wird.

In der Regel muss dann außer der aus Hygienegründen ohnehin notwendigen Auswechslung der Blende pro Patient kein weiteres Nachfüllen des Vorratsbehälters erfolgen. Falls dies bei einem Patienten doch notwendig ist, wird einfach die komplette Blende ausgetauscht, und durch eine neue Blende mit gefülltem Vorratsbehälter ersetzt.

Vorzugsweise wird das Glasfaserkabel an wenigstens einem, vorzugsweise an beiden Enden mit Endstücken, sogenannten Konnektoren, ausgestattet, die ein sehr einfaches Auswechseln des Glasfaserkabels im Falle der Verschmutzung oder Beschädigung ermöglichen, indem das Glasfaserkabel mit einem entsprechenden Endstück auf einfache Art und Weise an die Laserquelle auf der einen Seite und/oder das Handstück an der anderen Seite angekoppelt werden kann durch einfaches Einstecken, Einrasten oder Ähnliches. Eine separate Justierung des Glasfaserkabels ist nicht mehr notwendig bzw. besteht lediglich in einer einfachen, vor allem mechanischen, Justierung bzw. Zentrierung dieses Endstückes gegenüber dem entsprechenden Ansatzteil.

Hierfür kommen insbesondere Fiber-Konnektoren des Typs SMA905, ST und FC, in Frage.

Als Glasfaser wird vorzugsweise eine Multimode-Glasfaser verwendet, um Licht unterschiedlicher Wellenlängen einkoppeln zu können, also das Glasfaserkabel ggf. in unterschiedliche Varianten von Geräten einsetzen zu können

### c) Ausführungsbeispiele

Eine Ausführungsform gemäß der Erfindung ist im folgenden anhand der Figuren beispielhaft näher beschrieben. Es zeigen:
- Fig. 1:: Eine perspektivische Prinzipdarstellung des Handstücks,
- Fig. 2:: einen teilweisen Längsschnitt durch das Handstück gemäß Fig. 1,
- Fig. 3:: eine demgegenüber abgewandelte Befestigung der Blende,
- Fig. 4:: eine andere Version einer Detaildarstellung aus Fig. 2, und
- Fig. 5:: die Einteilung einer zu behandelnden Hautpartie in einzelne Bestrahlungsflächen.

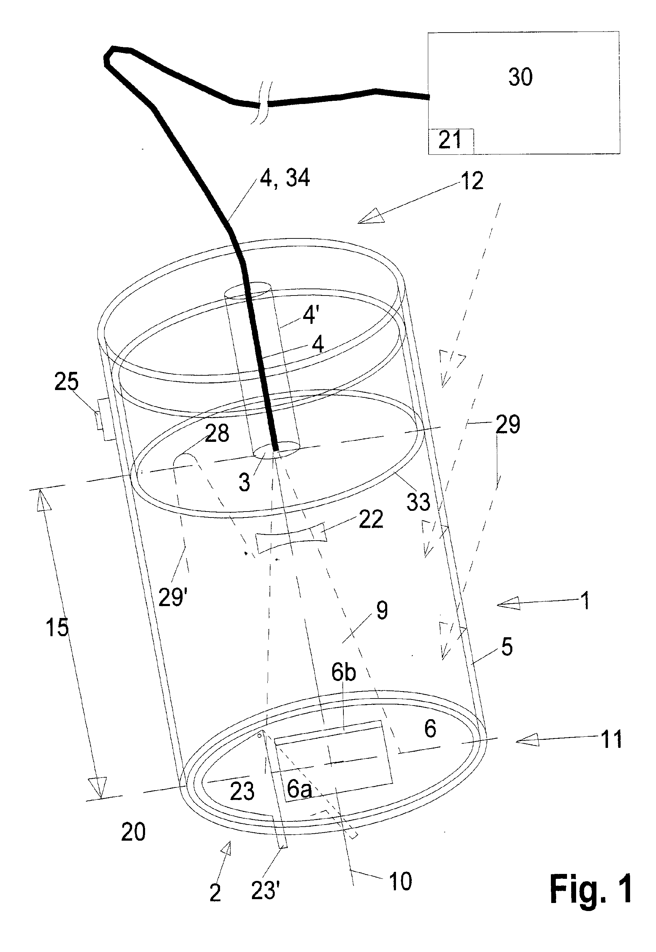

Fig. 1 zeigt eine primär aus durchsichtigem Acrylglas ("Plexiglas") gefertigtes Handstück 1, weshalb auch die rückwärtig liegenden Kanten erkennbar sind.

Das Handstück 1 umfasst als Abstandshalter 5 einen Rohrabschnitt mit rundem Querschnitt, der zugleich als Grundkörper des Handstückes dient. Am vorderen Ende 11 des Abstandshalters 5 ist an dessen Stirnfläche eine Blende 6 angeordnet.

Nahe dem hinteren Ende 12 ist im Abstandshalter 5 ein Haltekörper 33 befestigt, in dem das Ende einer Führungsvorrichtung 4 für die Zuführung von Behandlungslicht, insbesondere dessen Endstück 4', befestigt ist.

Aus dem vorderen Ende des Endstückes 4' und damit auch des Haltekörpers 3 strahlt das Austrittsende 3 dieser Führungsvorrichtung 4 das Behandlungslicht in Form eines Strahles 9 primär in Längsrichtung 10, also in Richtung auf das vordere Ende 11 und die dort angeordnete Blende 6, ab.

Von dem Endstück 4' führt der flexible Teil der Licht-Führungsvorrichtung 4, in der Regel ein Glasfaserkabel 34, zu der entfernt stehenden Lichtquelle 30 für das Behandlungslicht, in der Regel einem Excimer-Laser. In der Regel ist in Baueinheit mit der Lichtquelle auch die Steuerung 21 für das Bestrahlungsgerät angeordnet.

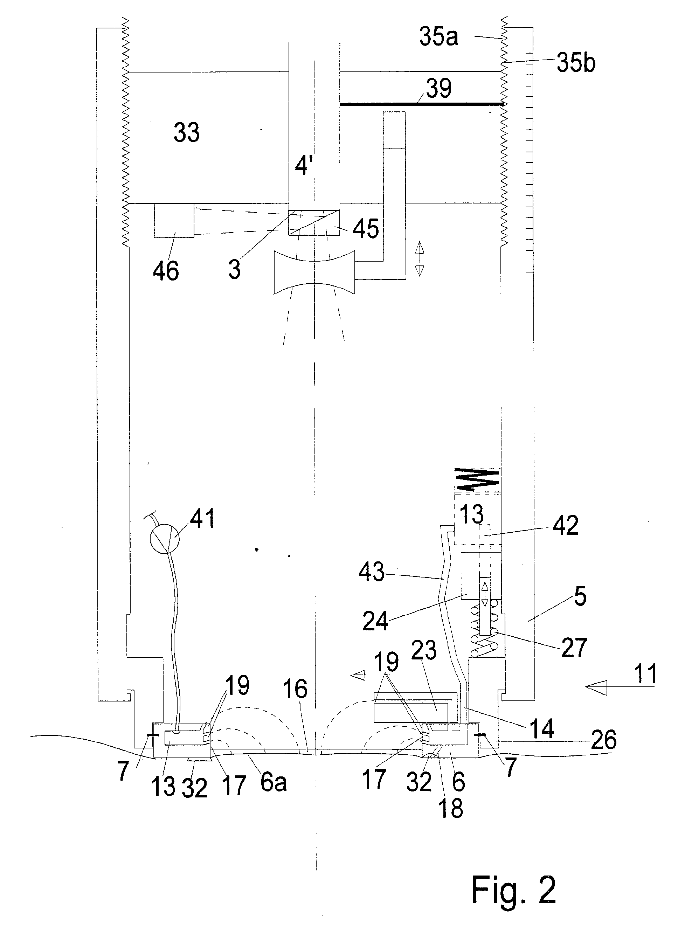

Das Endstück 4' der Führungsvorrichtung 4 ist insbesondere mittig, also auf der Längsachse, des Haltekörpers 33 angeordnet. Dieser ist mit seinem Außenumfang entlang des Innenumfanges des Abstandshalters 5 geführt und kann dort an unterschiedlichen Längspositionen festgelegt werden, beispielsweise durch ein insbesondere selbsthemmend ausgebildetes Gewinde zwischen dem Haltekörper 33 und dem Abstandshalter 5, wie in Detail in Fig. 2 dargestellt.

Dadurch kann der Abstand 15 zwischen dem Austrittsende 3 der Führungsvorrichtung 4 für das Behandlungslicht und der Blende 6 und damit der zu behandelnden Haut 20 des Patienten verstellt werden.

Im Strahlengang des Strahls 9 dazwischen befindet sich eine Streulinse 22, um die gewünschte Strahlverbreiterung zwischen Austrittsende 3 und dem vorderen Ende 11 des Abstandshalter 5 zu bewirken oder zu verstärken.

Am Außenumfang des tubusförmigen Abstandshalters 5 ist ein Handschalter 25 zum Einschalten der - in der Regel gepulsten - Lichtquelle 30 angeordnet.

Die Blende 6 am vorderen Ende 11 des Handstückes 1 ist ebenfalls wie der Abstandshalter 5 rund ausgebildet, und weist - vorzugsweise in der Mitte - eine möglichst große rechteckige Blendenöffnung 6a auf. Diese kann mit Hilfe einer oder mehrerer Abdeckungen 23, von denen in Fig. 1 nur beispielhaft eine auf einer Seite der rechteckigen Blendenöffnung 6a dargestellt ist, mehr oder weniger mittels Abdecken verkleinert werden. Die Abdeckungen 23 weisen zu diesem Zweck einen nach außen ragenden Handgriff 23' auf.

Das Handstück 1 wird mit seinem vorderen Ende, also in diesem Fall der Blende 6, auf die zu behandelnde Haut 20 des Patienten aufgesetzt, so dass die Blendenöffnung 6a die Behandlungsfläche darstellt.

Auch nach Aufsetzen des Handstückes 1 auf die Haut des Patienten ist diese Behandlungsfläche, also innerhalb der Blendenöffnung 6a, einsehbar aufgrund der Durchlässigkeit des tubusförmigen Abstandshalters 5 für Tageslicht 29 oder ein speziell hierfür eingesetztes Orientierungslicht 29'.

Eine solche Lichtquelle 28 für Orientierungslicht 29' kann stattdessen - beispielsweise in Form einer Leuchtdiode - auch direkt im Inneren des Handstückes 1, vorzugsweise im Freiraum zwischen dem Haltekörper 33 und der Blende 6, angeordnet sein und strahlt das Orientierungslicht 29' zumindest auf den Bereich der Blendenöffnung 6a ab.

Fig. 2 zeigt einen Längsschnitt durch eine der möglichen Bauformen, wie sie in Fig. 1 prinzipiell dargestellt ist:

Im hinteren Teil des tubusförmigen Abstandshalters 5 weist dieser ein Innengewinde 35a auf, wie in der rechten Bildhälfte dargestellt, in welchem der Haltekörper 33, der auf seinem Außenumfang ein entsprechendes Außengewinde 35b trägt, verschiebbar ist. Dadurch wird zwar auch die mittig im Haltekörper 33 befindliche Führungsvorrichtung 4 und deren Endstück 4' mitgedreht, was jedoch in der Praxis dazu führt, dass das Endstück in Ruhe gehalten und gegenüber dem Rest des Handstückes verdreht wird. Wegen der vorzugsweise zentralen Anordnung der Austrittsöffnung 3 und damit des Endstückes 4' ist das Verdrehen unproblematisch. Auch eine längsfeste, drehbare Lagerung des Endstückes 4' im Haltekörper 33 ist möglich.

Die Längsverlagerung des Haltekörpers 33 kann auch auf andere Art und Weise und ohne relative Verdrehung zueinander erfolgen, beispielsweise durch die in der linken Bildhälfte der Fig. 2 dargestellte Längsverschiebbarkeit mittels z. B. stufenweiser Arretierung, indem beispielsweise ein federnd vorgespannter Arretierstift 36, der radial verschiebbar im Haltekörper fixiert ist, radial nach außen bis zum formschlüssigen Eindringen in eine entsprechende Arretierbohrung 37 einer in Längsrichtung 10 verlaufenden Bohrungsreihe eingreift. Der Arretierstift 36 ist vorzugsweise mittels Federkraft in die verriegelte Position vorgespannt und muss von Hand demgegenüber zurückgezogen werden zum Verstellen des Haltekörpers 33 in Längsrichtung.

Die Einstellung des Abstandes 15 kann selbst oder in Form ihrer Auswirkung auf die Bestrahlungsintensität pro Flächeneinheit an der Längsposition der Blendenöffnung 6a entweder - wie in der rechten Bildhälfte dargestellt - an einer Skala 38 abgelesen werden, indem diese Skala 38 in Längsrichtung 10 entlang der Außenfläche des Abstandshalters 5 aufgebracht ist und wegen deren durchsichtigem Material eine vorzugsweise ringförmig umlaufende Markierung 39 auf dem Außenumfang des Haltekörpers 33 dessen Längsposition anzeigt.

Dagegen ist in der linken Bildhälfte der automatische Abgriff der Längsposition mittels eines elektrischen oder elektromechanischen Sensors 40 dargestellt, in dem einerseits ein elektrischer Kontaktpunkt an einer Längsposition und andererseits demgegenüber ein in Längsrichtung verlaufender Abgriff-Streifen, von denen einer auf dem Haltekörper 33 und der andere auf dem Abstandshalter 5 aufgebracht ist, zueinander in Kontakt geraten und über elektrische Leitungen mit dem Steuergeräte verbunden sind. Bei einer Verschraubungslösung wie in der rechten Bildhälfte muss auch der elektrische Kontaktpunkt umlaufend über den gesamte Umfang in Form eines elektrischen Kontaktringes ausgebildet sein.

Weiterhin kann durch Längsverstellung des die Linse 22 tragenden Linsenhalters 44 relativ zum Haltekörper 33 der Strahlquerschnitt in einem definierten Abstand, z. B. an der Blende 6 und damit die Bestrahlungsintensität, verstellt werden.

Zusätzlich ist am Haltekörper 33 eine Energiemeß-Vorrichtung, vorzugsweise fest, angeordnet:

Dabei ist unmittelbar vor dem Austrittsende 3 des Endstückes 4' ein optisches Element zum Auskoppeln eines Teils des Laserlichts aus dem Strahlengang in Richtung Blendenöffnung 6a angeordnet, beispielsweise ein teildurchlässiger Spiegel 45. Der Großteil des Laserlichts durchläuft die schräg im Strahlengang stehende Spiegelfläche ohne Reflexion, ein definierter kleiner Anteil von 1-10% des Laserlichts wird von der Spiegelfläche jedoch reflektiert und dadurch um ca. 90° seitlich abgelenkt auf einen ebenfalls am Haltekörper 33 befindlichen Meßkopf 46, der die Energiedichte des abgelenkten Teilstrahles ermittelt, aus dem auf die Energiedichte des Gesamtstrahles an der Stelle des auskoppelnden Elementes, also des teildurchlässigen Spiegels 45, geschlossen werden kann.

Das auskoppelnde Element kann auch aus dem Strahlengang herausbewegbar ausgebildet sein, um nicht während, sondern nur z. B. vor einer Behandlung eine Referenzmessung durchzuführen, während der Behandlung jedoch auf der zu behandelnden Fläche die volle Energiedichte zur Verfügung zu haben.

Fig. 2 zeigt im unteren Bereich Detail-Ausbildungen der Blende 6 und der darin befindlichen Blendenöffnung 6a.

Dabei ist die Blende 6 nicht direkt am unteren Ende 11 des tubusförmigen Abstandshalters 5 befestigt, sondern in einem ebenfalls vorzugsweise ringförmigen Blendenhalter 26 aufgenommen. Der Blendenhalter 26 seinerseits ist in Längsrichtung 10 verschiebbar im vorderen Ende 11 des Abstandshalters 5 geführt und steht vorzugsweise über dessen vorderes stimseitiges Ende vor.

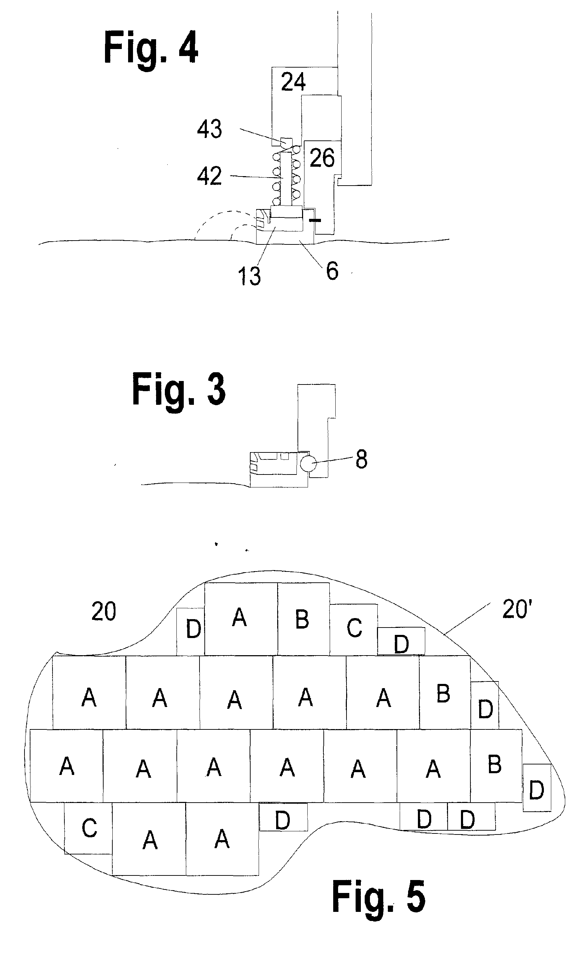

Ebenso steht die Blende 6 selbst stirnseitig über das vordere freie Ende des Blendenhalters 26 vor. Die Blende 6 ist gegenüber dem Blendenhalter 26 leicht lösbar, beispielsweise mittels eines oder mehrerer quer zur Längsrichtung einschiebbarer Verriegelungsteile 7, wie in Fig. 2 dargestellt vorzugsweise formschlüssig arretierbar. Als Verriegelungsteil kann insbesondere ein O-Ring 8 dienen, wie in Fig. 3 dargestellt, der mit seinem Querschnitt zur Hälfte in einer rinnenförmigen Aussparung des Außenumfanges der Blende 6 und zur anderen Hälfte in einer analogen rinnenförmigen Aussparung im Innenumfang des Blendenhalters 26 formschlüssig fixiert ist.

Der Blendenhalter 26 ist mittels einer Druckfeder 27 in die bezüglich des Abstandshalters 5 ausgeschobene Position vorgespannt und stützt sich vorzugsweise gegenüber dem in Längsrichtung beweglichen Taster eines Schalters 24 ab, der am Innenumfang des Abstandshalters 5 befestigt ist. Durch Aufdrücken des Handstückes mittels der Blende 6 auf der Haut 20 eines Patienten wird Blende 6 mit Blendenhalter 26 zusammen in Längsrichtung bezüglich des Abstandshalters 5 nach innen verschoben und damit der Schalter 24 aktiviert, was ein Signal für das Aufbringen einer ausreichenden Anspresskraft gegenüber der Haut der Patienten ergibt.

Zu diesem Zweck stützt sich vorzugsweise die Blende 6 auch gegenüber einer entsprechenden Schulter im Blendenhalter 26 an dieser ab, so dass die Druckkraft nicht vollständig durch das Verriegelungsteil 7 bzw. dem O-Ring 8 übertragen werden muss.

Fig. 2 zeigt ferner in der linken Bildhälfte nur an der Blende 6, in der rechten Bildhälfte auch an einer parallel zur Blende 6 verschiebbaren Abdeckung 23 für die Blendenöffnung 6a Austrittsöffnungen 18 bzw. 19 für eine Flüssigkeit.

Die Austrittsöffnungen sind dabei in Richtung auf die Blendenöffnung 6a, also die Behandlungsöffnung gerichtet und befinden sich teils in den Flanken 17 der Blendenöffnung und/oder auch in deren ins Innere des Handstückes 1 hineingerichteten Oberseite, um durch bogenförmiges Aussprühen auch den mittleren Bereich der Blendenöffnung erreichen zu können.

Bei auf dieser Oberseite laufender Abdeckung 23 sind dort vorhandene Austrittsöffnungen 19 nicht sinnvoll.

Handelt es sich bei der auszubringenden Substanz nicht um eine Behandlungssubstanz sondern um eine reine Markierungssubstanz, so befinden sich Austrittsöffnungen 18 - ebenfalls wieder vorzugsweise umlaufend um die Blendenöffnung 6a - auf der der Haut 20 des Patienten zugewandten Unterseite der Blende 6, wie gestrichelt in der rechten Bildhälfte dargestellt.

Stattdessen kann das Aufbringen einer Markierungsflüssigkeit im Randbereich um die Blendenöffnung 6a herum auch mittels eines Stempels 32 erfolgen, der in Form eines rechteckig umlaufenden Stempelkissens auf der Unterseite der Blende um die Blendenöffnung 6a herum angeordnet ist, wie in der linken Bildhälfte der Fig. 2 ersichtlich.

Auch für das Aufbringen des notwendigen Druckes zum Ausbringen einer Substanz aus den Austrittsöffnungen 18 bzw. 19 stehen mehrere Möglichkeiten zur Verfügung.

In der linken Bildhälfte ist der Vorratsbehälter 13 direkt innerhalb der Blende 6 untergebracht. Der notwendige Auspressdruck wird jedoch durch eine Pumpe 41 erzeugt, die abseits der Blende 6, beispielsweise im hohlen Inneren des Abstandshalters 5, angeordnet und mit dem Vorratsbehälter 13 über eine Schlauchleitung verbunden ist. Dadurch kann in einem per Membran im Inneren des Vorratsbehälters 13 abgetrennten Luftraum pneumatisch der notwendige Austriebsdruck erzeugt werden.

Die Pumpe 41 kann auch außerhalb des Handstückes 1 angeordnet sein, ebenso wie auch der Vorratsbehälter 13.

Die rechte Bildhälfte zeigt eine Lösung, bei der der Austreibdruck mechanisch erzeugt wird, indem ein in Längsrichtung verlagerbarer Pressstempel 42 sich in den Vorratsbehälter 13 hineinverlagert, dessen durch Verbrauch abnehmendes Volumen dann durch eine Federvorspannung nachreguliert wird. Der Vorratsbehälter 13 kann sich ebenfalls an der Innenseite des Abstandshalter 5 befinden, und der Presstempel 42 zusammen mit dem Schalter 24 und dessen Taster oder mittels dieses Tasters in Längsrichtung beim Aufsetzten des Handstückes auf die Haut des Patienten bewegt werden. Der Vorratsbehälter 13 steht dann über eine Leitung 31 mit den Austrittsöffnungen in der Blende 6 bzw. in der oder den Abdeckungen 23 in Verbindung.

Fig. 4 zeigt eine dahingehend abgewandelte Detaillösung, dass der Pressstempel 42 einerseits in den direkt in der Blende 6 angeordneten Vorratsbehälter 13 in Längsrichtung von hinten her eintauchen kann, andererseits jedoch soweit nach hinten verlängert ist, dass er - ohne Kraftbeaufschlagung und mittels Federkraft abgehoben - unter Überwindung der Kraft der Feder einen z. B. elektrischen Kontaktpunkt 43 des Schalters 24 kontaktiert.

Dies ergibt eine besonders einfache Bauform aufgrund Funktionsvereinigung von Pressstempel und Schalterelementen und vermeidet eine elektrische Energieversorgung für eine Pumpe zur Erzeugung des notwendigen Druckes für eine aufzubringende Flüssigkeit.

Weiterhin zeigt Fig. 5 die Vorgehensweise beim Bestrahlen einer zu behandelnden Freiformfläche 20' der Haut 20.

Dabei wird die Blendenöffnung 6 des Handstückes zunächst auf maximale Größe eingestellt. Die Grundform der Blendenöffnung 6a sei ein Quadrat, also ein Rechteck, die in der Fig. 5 als Einzelflächen A dargestellt sind.

Diese Einzelflächen A der maximalen Blendenöffnung werden innerhalb der Freiformfläche 20' so ofr als möglich unmittelbar aneinander anschließend, also ohne Zwischenräume und ohne Überlappungen, nebeneinander angeordnet, vorzugsweise in Form gerade, hinsichtlich der Außenkanten fluchtender, Reihen, und unter Zurücklassung so geringer Restflächen wie möglich.

Anschließend wird die Blendenöffnung 6a vorzugsweise in nur einer Flächenrichtung verkleinert zu einer dann nicht mehr quadratischen Rechtecksfläche B.

Diese Einzelfläche B wird - wiederum durch entsprechendes Aufsetzen des Handstückes unter entsprechender Positionierung der Blendenöffnung - auf die verbleibenden Restflächen so oft als möglich und wiederum unmittelbar aneinander angrenzend angeordnet mit dem Ziel, die wiederum verbleibenden, bisher unbehandelten Restflächen so gering wie möglich zu halten.

Diese Restflächen werden mit wiederum verkleinerten Einzelflächen C besetzt, die dann in der Regel durch Verkleinerung der bisherigen Blendenöffnung insbesondere in der anderen, bisher nicht veränderten, Flächenrichtung der Blendenöffnung gestaltet werden, so daß die Teilflächen c wiederum quadratisch, jedoch deutlich kleiner als die Teilflächen A, sein können.

In dieser Weise wird fortgefahren, bis keine Restflächen mehr vorhanden sind bzw. die verbleibenden Restflächen zu gering sind, um in der vorbeschriebenen Weise flächig weiterbehandelt zu werden.

### BEZUGSZEICHENLISTE

- 1: Handstück
- 2: Kontaktfläche
- 3: Austrittsende
- 4: Laserstrahl-Führungsvorrichtung
- 4': Endstück der Führungsvorrichtung
- 5: Abstandshalter
- 6: Blende
- 6a: Blendenöffnung
- 6b: umgebende Rande
- 7: Verriegelungsteil
- 8: O-Ring
- 9: Strahl
- 10: Längsrichtung
- 11: vorderes Ende
- 12: hinteres Ende
- 13: Vorratsbehälter
- 14: Bohrung
- 15: Abstand
- 16: Behandlungssubstanz
- 17: Flanken
- 18: Austrittsöffnung
- 19: Austrittsöffnung
- 20: Haut
- 21: Steuerung
- 22: Streulinse
- 23: Abdeckungen
- 23': Handgriff
- 24: Schalter
- 25: Handschalter
- 26: Blendenhalter
- 27: Druckfeder
- 28: Lichtquelle
- 29: Tageslicht
- 29': Orientierungslicht
- 30: Lichtquelle
- 31: Leitung
- 32: Stempel
- 33: Haltekörper
- 34: Glasfaserkabel
- 35a: Innengewinde
- 35b: Außengewinde
- 36: Arretierstift
- 37: Arretierbolzen
- 38: Skala
- 39: Markierung
- 40: Sensor
- 41: Pumpe
- 42: Preßstempel
- 43: Kontaktpunkt
- 44: Linsenhalter
- 45: teildurchlässiger Spiegel
- 46: Meßkopf

## Patentansprüche

1. Dermatologisches Handstück (1) für die Bestrahlung von lebender Haut mit Behandlungslicht aus einer vom Handstück (1) entfernten Lichtquelle, insbesondere einer Laser-Lichtquelle (30), mit
- einer Kontaktfläche (2) am vorderen Ende (11) des Handstückes zum Kontaktieren der zu behandelnden Haut (20),
- einem Austrittsende (3) der Laserstrahl-Führungsvorrichtung (4) im Handstück (1) und
- Abstandshalter (5) zwischen dem Austrittsende (3) und der Kontaktfläche (2)
**dadurch gekennzeichnet, dass**
- der Abstandshalter (5) den Strahl (9) des Behandlungslichts wenigstens zwischen Austrittsende (3) und Kontaktfläche (2) allseitig umgibt und
- aus einem für das Behandlungslicht nicht durchlässigen, jedoch für wenigstens ein Orientierungslicht mit anderer Wellenlänge, insbesondere Tageslicht (29), durchlässigen Material besteht.

2. Dermatologisches Handstück (1) für die Bestrahlung von lebender Haut mit Behandlungslicht aus einer vom Handstück (1) entfernten Lichtquelle, insbesondere einer Laser-Lichtquelle (30), mit
- einer Kontaktfläche (2) am vorderen Ende (11) des Handstückes (1) zum Kontaktieren der zu behandelnden Haut (20),
- einem Austrittsende (3) der Laserstrahl-Führungsvorrichtung (4) im Handstück (1) und
- Abstandshalter (5) zwischen dem Austrittsende (3) und der Kontaktfläche (2)
**dadurch gekennzeichnet, dass**
- die Kontaktfläche (2) eine Blende (6) umfasst, die außerhalb der Blendenöffnung (6a) aus einem für das Behandlungslicht nicht durchlässigen, jedoch für wenigstens ein Orientierungslicht mit anderer Wellenlänge, insbesondere Tageslicht (29), durchlässigen Material besteht.

3. Dermatologisches Handstück (1) nach Anspruch 1 oder 2,
**dadurch gekennzeichnet, dass**
die Lichtquelle ein Excimer-Laser ist und das Behandlungslicht eine Wellenlänge von 308 nm besitzt.

4. Dermatologisches Handstück (1) nach einem der vorherigen Ansprüche,
**dadurch gekennzeichnet, dass**
der Strahl (9) stromabwärts des Austrittsendes (3) eine definierte Strahldivergenz aufweist, die insbesondere durch eine im Strahlengang stromabwärts des Austrittsendes (3) angeordnetes optisches Element, insbesondere eine Streulinse (22), bewirkt wird.

5. Dermatologisches Handstück (1) nach einem der vorherigen Ansprüche,
**dadurch gekennzeichnet, dass**
die Länge des Abstandes (15) zwischen Austrittsende (3) und Kontaktfläche (2) in Längsrichtung (10) verstellbar, insbesondere stufenlos verstellbar, ist.

6. Dermatologisches Handstück (1) nach einem der vorherigen Ansprüche,
**dadurch gekennzeichnet, dass**
- die Blende (6) Bestandteil des Abstandshalters (5) ist, und insbesondere
- die Größe der Blendenöffnung (6a) veränderbar, insbesondere stufenlos veränderbar ist, und insbesondere
- die Blende (6) am Abstandshalter (5) auswechselbar ist.

7. Dermatologisches Handstück (1) nach einem der vorherigen Ansprüche,
**dadurch gekennzeichnet, dass**
- der Abstandshalter (5) und/oder die Blende (6) aus für Tageslicht durchlässigem Acrylglas, insbesondere klarem Acrylglas, besteht, und insbesondere
- der Abstandshalter (5) die Form eines Rohrabschnittes mit geschlossenem, insbesondere rundem, Querschnitt besitzt.

8. Dermatologisches Handstück (1) nach einem der vorherigen Ansprüche,
**dadurch gekennzeichnet, dass**
die Länge des Abstandes (15) zwischen dem Austrittsende (3) und der Kontaktfläche (2) verstellt wird durch Änderung der Längsposition des Austrittsendes (3) relativ zum Abstandshalter (5), insbesondere durch Verschrauben entlang des Abstandshalters (5).

9. Dermatologisches Handstück (1) nach einem der vorherigen Ansprüche,
**dadurch gekennzeichnet, dass**
die Blendenöffnung (6a) rechteckig, insbesondere quadratisch, oder sechseckig ist.

10. Dermatologisches Handstück (1) nach einem der vorherigen Ansprüche,
**dadurch gekennzeichnet, dass**
die Kontaktfläche (2), insbesondere die Blende (6), als - insbesondere elektrischer oder mechanischer - Schalter (24) ausgebildet ist, insbesondere als Tastschalter durch Relativbewegung der Kontaktfläche in Längsrichtung (10) gegenüber dem Abstandshalter (5).

11. Dermatologisches Handstück (1) nach einem der vorherigen Ansprüche,
**dadurch gekennzeichnet, dass**
der Schalter eine Sperre zum Aktivieren der Lichtquelle freigibt und insbesondere die Lichtquelle direkt aktiviert.

12. Dermatologisches Handstück (1) nach einem der vorherigen Ansprüche,
**dadurch gekennzeichnet, dass**
eine Lichtquelle für Orientierungslicht (29') im Handstück (1) integriert ist und die Kontaktfläche (2) beleuchtet.

13. Dermatologisches Handstück (1) nach einem der vorherigen Ansprüche,
**dadurch gekennzeichnet, dass**
der die Blendenöffnung (6a) umgebenden Rand (6b) der Blende (6) Austrittsöffnungen (18) für ein flüssiges oder pastöses Markierungsmittel umfasst, die gegen die zu behandelnde Haut (20) gerichtet sind zur Markierung des Randes der Blendenöffnung (6a), insbesondere des Randes außerhalb der Blendenöffnung (6a).

14. Dermatologisches Handstück (1) nach einem der vorherigen Ansprüche,
**dadurch gekennzeichnet, dass**
die Blende (6) nahe ihrer Blendenöffnung (6a), insbesondere in deren Flanken (17), Austrittsöffnungen (19) aufweist, die so angeordnet sind, dass damit die gesamte Blendenöffnung (6a) mit einer flüssigen oder pastösen Behandlungssubstanz (16) benetzt werden kann.

15. Dermatologisches Handstück (1) nach einem der vorherigen Ansprüche,
**dadurch gekennzeichnet, dass**
die Austrittsöffnungen (19) mittels einer Steuerung (21) wahlweise vor, während und/oder nach der Bestrahlung der Blendenöffnung (6a) mit Behandlungslicht geöffnet werden und die Benetzung der Haut mit der Behandlungssubstanz (16) erfolgt.

16. Dermatologisches Handstück (1) nach einem der vorherigen Ansprüche,
**dadurch gekennzeichnet, dass**
die Blende (6) über wenigstens eine Leitung (31), insbesondere über Bohrungen (14) mit dem Vorratsbehälter (13) für die Behandlungssubstanz (16) in Verbindung steht, die am oder im Abstandshalter (5), insbesondere im Inneren dessen Wandung, oder innerhalb dessen freien Querschnittes, angeordnet ist.

17. Dermatologisches Handstück (1) nach einem der vorherigen Ansprüche,
**dadurch gekennzeichnet, dass**
der Vorratsbehälter (13) für die Behandlungssubstanz (16) in oder an der auswechselbaren Blende (6) angeordnet ist und das Austreten der Behandlungssubstanz (16) bzw. Markierungssubstanz durch die Austrittsöffnungen (18 bzw. 19) durch den Druck auf die Blende (6) erfolgt, welcher gleichzeitig die als Schalter (24) ausgebildete Blende (6) schaltet.

18. Dermatologisches Handstück (1) nach einem der vorherigen Ansprüche,
**dadurch gekennzeichnet, dass**
die Höhe des aufzubringenden Druckes einstellbar ist, insbesondere durch Einstellbarkeit der Vorspannung einer dem Gegendruck zur Verfügung stellenden Feder, insbesondere Druckfeder (27).

19. Dermatologisches Handstück (1) nach einem der vorherigen Ansprüche,
**dadurch gekennzeichnet, dass**
im Strahlengang des Behandlungslichts innerhalb des Handstückes, insbesondere aus diesem entfernbar, eine Energie-Meßvorrichtung zum Messen der Strahlenergie, insbesondere dessen Energiedichte, angeordnet ist.

20. Dermatologisches Handstück (1) nach Anspruch 19,
**dadurch gekennzeichnet, dass**
- die Energiemeßvorrichtung ein optisches Auskopplungselement zum Auskoppeln eines definierten Anteiles des Laserstrahles, insbesondere einen teildurchlässigen Spiegel (45), umfaßt sowie einen Meßkopf (46), auf den der ausgekoppelte Teilstrahl gerichtet ist, und insbesondere
- die Energiemeßvorrichtung unmittelbar stromabwärts des Austrittsendes (3) der Laserstrahlführungsvorrichtung (4) im Handstück (1), insbesiondere befestigt an dem Haltekörper (33), angeorndet ist oder nahe des vorderen Endes des Handstückes (1), insbesondere unmittelbar vor der Blende (6).

21. Verfahren zum Zusammensetzen einer Freiformfläche aus möglichst wenigen, hinsichtlich Form und Größe begrenzt wahlfreien Einzelflächen, insbesondere einer Behandlungsfläche aus verkleinerbaren Blendenöffnungen (6a),
**dadurch gekennzeichnet, dass**
- die Einzelflächen einfache geometrische Formen mit geraden Außenkanten besitzen und
- zunächst Einzelflächen (A) maximaler Größe und gleicher Form so oft als möglich aneinander anschließend in der Freiformfläche angeordnet werden,
- anschließend in den Restflächen demgegenüber verkleinerte, insbesondere in nur einer Flächenrichtung verkleinerte, Einzelflächen (B) so oft als möglich und insbesondere aneinander anschließend angeordnet werden, und
- der letztgenannte Verfahrensschritt bei Bedarf ein- oder mehrfach wiederholt wird, und insbesondere
- die maximal große Einzelfläche Rechteckform, insbesondere Quadratform, besitzt.

22. Verfahren nach einem der vorhergehenden Verfahrensansprüche,
**dadurch gekennzeichnet, dass**
bei Verkleinerung der Einzelflächen von der maximalen Größe aus die geometrische Form der Einzelfläche, also Rechteck, Dreieck, Trapez, unverändert bleibt.
